# DEMANDE DE BREVET EUROPEEN

(11) **EP 0 573 718 A1**
(43) Date de publication de la demande: **15.12.1993**
(21) Numéro de dépôt: 92401599.3
(22) Date de dépôt: 10.06.1992
(51) Int. Cl.: E02D 1/04, E21B 7/02

(54) **Installation mobile pour le prélèvement automatique d'échantillons de sol**

(71) Demandeur: Eloy, Michel, F-62170 Brimeux (FR)
(72) Inventeur: Eloy, Michel, F-62170 Brimeux (FR)
(74) Mandataire: Descourtieux, Philippe

(57) **Abrégé**

L'installation comporte un véhicule léger (2) par exemple un véhicule tout terrain à pneus larges basse pression, de 250 kg environ; la sonde (13) de prélèvement d'échantillons de sol est montée à l'extrémité d'un bras porteur (18) pivotant autour d'un axe (17) solidaire du châssis du véhicule (2); de plus un ensemble compensateur (21), par exemple au moins une roue (24,25) supportée par un bras, est solidaire du châssis et apte à s'appliquer sur le sol pendant le prélèvement en sorte d 'assurer la stabilité du véhicule.

## Description

La présente invention concerne une installation mobile spécialement conçue pour effectuer automatiquement les prélèvements d'échantillons de sol, destinés aux analyses du sol ou de la terre, en particulier dans le domaine de l'agriculture.

Les analyses de la couche superficielle du sol sont indispensables en agriculture pour prévoir les traitements qui seront ultérieurement nécessaires notamment l'apport d'engrais selon des dosages adaptés.

Pour effectuer ces analyses , il est indispensable de prélever des échantillons du sol, en nombre suffisant pour une parcelle donnée , pour que le résultat obtenu sur le mélange des échantillons soit représentatif , par exemple une vingtaine d'échantillons pour une parcelle de dix hectares.

Une telle prise d'échantillons est effectuée le plus souvent manuellement à l'aide d'une sonde ou tarrière.

On a déjà proposé de rendre automatique le prélèvement d'échantillons , soit de manière continue dans le document FR.A.2.462.697 soit de manière intermittente dans le document FR.A.2.562.665.

Ce dernier document décrit un appareil de prélèvement automatique d'échantillons de sol qui comprend un élément formant sonde de prélèvement , un dispositif approprié d'entraînement en rotation de cet élément formant sonde , un mécanisme de déplacement en translation sensiblement verticale , par rapport au bâti de l'appareil , du dispositif d'entraînement et de l'élément formant sonde,et des moyens de commande sélective du mécanisme de déplacement.

Il est prévu , dans un mode de réalisation , que le bâti de cet appareil de prélèvement peut être monté sur un véhicule approprié de manière à permettre la récupération des échantillons sans descendre de véhicule.

A la connaissance du demandeur , les appareils du type décrit ci-dessus sont toujours montés sur des tracteurs, c'est-à-dire des véhicules lourds , spécialement conçus pour se déplacer dans les champs . Ceci présente différents inconvénients. Le passage du tracteur provoque un tassement du sol , concrétisé par les traces de roues , qui peut être gênant. L'utilisation du tracteur n'est pas possible dans toutes les conditions météorologiques.

De plus l'appareil décrit dans le document FR.A.2.562.665 ne permet de prélever que dans des zones directement accessibles par le véhicule.

Le premier but que s'est fixé le demandeur est de proposer une installation mobile pour le prélèvement automatique d'échantillons de sol qui pallie les inconvénients constatés.

Ce but est parfaitement atteint par l'installation mobile de l'invention qui comporte de manière connue un véhicule sur lequel est montée une sonde de prélèvement entraînable en rotation et en translation verticale. De manière caractéristique le véhicule étant un véhicule léger, la sonde est montée à l'extrémité d'un bras porteur pivotant autour d'un axe solidaire du châssis du véhicule , de plus l'installation mobile de l'invention comprend un ensemble compensateur , solidaire du châssis du véhicule et apte à s'appliquer sur le sol au moins pendant le prélèvement en sorte d'assurer la stabilité du véhicule.

Ainsi le bras porteur , monté pivotant , permet en déplaçant la sonde à une certaine distance du véhicule d'effectuer des prélèvements dans des zones qui ne sont pas directement accessibles du véhicule. La légèreté du véhicule évite l'écrasement du sol et lui confère plus de maniabilité. L'ensemble compensateur permet d'assurer la stabilité du véhicule , lors du pivotement du bras et de l'actionnement des moyens de déplacement de la sonde, malgré la légèreté du véhicule.

Le véhicule léger est de préférence un véhicule tout terrain à quatre roues motrices, ayant des pneus larges basse pression dont le poids normal est de l'ordre de 250kg. Un tel véhicule est utilisable par tout temps , même avec la surcharge due aux autres équipements de l'invention.

Selon le mode préféré de l'invention, l'ensemble compensateur comprend au moins une roue complémentaire , non motrice, montée à l'extrémité libre d'un bras solidaire du châssis du véhicule et l'installation mobile comporte des moyens de pression, aptes à repousser ladite roue vers le sol.

Pour que soit assurée la stabilité du véhicule , la zone d'application sur le sol de l'ensemble compensateur et notamment de la roue précitée est extérieure au polygone normal de sustentation du véhicule et située du côté du bras porteur de la sonde.

Avantageusement l'installation mobile de l'invention comporte un bac de réception des échantillons qui est fixé sur le châssis du véhicule à une distance de l'axe de pivotement du bras porteur qui est sensiblement égale à la longueur dudit bras. De la sorte , les échantillons prélevés automatiquement peuvent être déposés dans le bac de réception après une simple rotation angulaire du bras porteur autour de son axe.

Un autre objet de l'invention est de proposer une installation mobile de prélèvement automatique d'échantillons qui puisse effectuer des prélèvements à grande profondeur , c'est-à-dire jusqu'à des profondeurs de l'ordre du mètre. Des prélèvements à 90 centimètres sont utiles en particulier pour les analyses de reliquats d'azote , comparativement aux analyses classiques dont les échantillons sont prélevés à 30 centimètres environ.

Ceci est obtenu grâce à un premier mode particulier de réalisation de l'installation mobile précitée de l'invention sur laquelle le bras porteur comporte :
a. une plaque support, horizontale , sur laquelle sont montés coulissants la sonde et au moins le moteur d 'entraînement en rotation de la sonde , b. une pièce de jonction sur laquelle est fixée rigidement ladite plaque support,
c. deux tiges parallèles , de même longueur , dont les extrémités sont solidaires d'une part du montant vertical constituant l'axe de pivotement du bras porteur et d'autre part de la pièce de jonction, de sorte que l'ensemble forme un parallélogramme déformable,
d. un vérin dont le corps est solidaire du montant vertical et dont l'extrémité de la tige du piston est solidaire d'une des deux tiges formant le parallélogramme déformable.

On comprend que l'action du vérin a pour effet de déplacer en hauteur la sonde et son moteur d'entraînement . Grâce à la disposition particulière de mise en oeuvre, la pièce de jonction qui forme le côté opposé au montant vertical dans le parallélogramme déformable reste constamment verticale et , par conséquent, la plaque support de la sonde et du moteur d'entraînement reste constamment horizontale. Ainsi la direction de la sonde lors du prélèvement reste toujours verticale.

Le déplacement vertical de la sonde peut aussi être obtenu grâce à un système du type vis-écrou. Ainsi dans un second mode particulier de réalisation, l'installation de l'invention comporte une tige filetée qui est entrainée en rotation par un moteur et montée selon l'axe de pivotement du bras porteur dans un montant vertical qui comporte une fente verticale ; de plus le bras porteur comporte une plaque support, horizontale, sur laquelle sont montés coulissants, vers une extrémité, la sonde et au moins le moteur d'entraînement en rotation de la sonde et dont l'autre extrémité, passant à travers le montant vertical à travers ladite fente, est solidaire d'un écrou traversé par la tige filetée. La rotation sur elle-même de la tige filetée provoque le déplacement vertical de l'écrou, de la plaque support et par conséquent de la sonde.

De préférence l'installation mobile selon l'invention comporte un vérin dit de pénétration dont le corps est fixé à l'extrémité libre du bras porteur, des glissières de maintien qui sont fixées verticales à l'extrémité libre du bras porteur et le long desquelles peuvent coulisser la sonde et son moteur d'entraînement en rotation ; de plus l'extrémité libre de la tige du vérin de pénétration est reliée par une pièce de liaison au moteur d'entraînement , en sorte que l'action du vérin provoque le déplacement vertical de la sonde dans les glissières de maintien.

Cette dernière disposition est particulièrement intéressante pour les prélèvements à grande profondeur, puisque le déplacement total de la sonde résulte d'une part, soit du déplacement angulaire (dans un plan vertical) du bras porteur selon le premier mode particulier de réalisation précité, soit du déplacement vertical de la plaque support sous l'action du système vis-écrou selon le deuxième mode particulier de réalisation, et d'autre part de l'action du vérin de pénétration . Ce cumul de deux organes pour réaliser un déplacement déterminé permet d'avoir soit dans le premier mode de réalisation précité, un déplacement angulaire du bras plus réduit, soit dans le deuxième mode de réalisation précité un déplacement vertical de l'écrou moins important, et dans les deux cas d'utiliser un vérin dont la tige du piston est plus courte.

L'invention sera mieux comprise à la lecture de la description qui va être faite du mode de réalisation préféré d'une installation mobile de prélèvement automatique d'échantillons , illustrée par le dessin annexé dans lequel :
La figure 1 est une représentation de dessus très schématique de l'installation comprenant un véhicule léger , un bras porteur et un compensateur à deux roues,
La figure 2 est une représentation schématique de côté du bras porteur, et de la sonde, selon le premier mode particulier de réalisation
La figure 3 est une représentation schématique de côté du compensateur à roue,
Les figures 4A et 4B sont deux représentations schématiques de l'extrémité du bras porteur équipé d'un vérin de pénétration, de côté (figure 4A) et de dessous (figure 4B).
La figure 5 est une vue schématique en section transversale de la sonde.
La figure 6 est une représentation schématique de côté du bras porteur et de la sonde selon le deuxième mode particulier de réalisation.

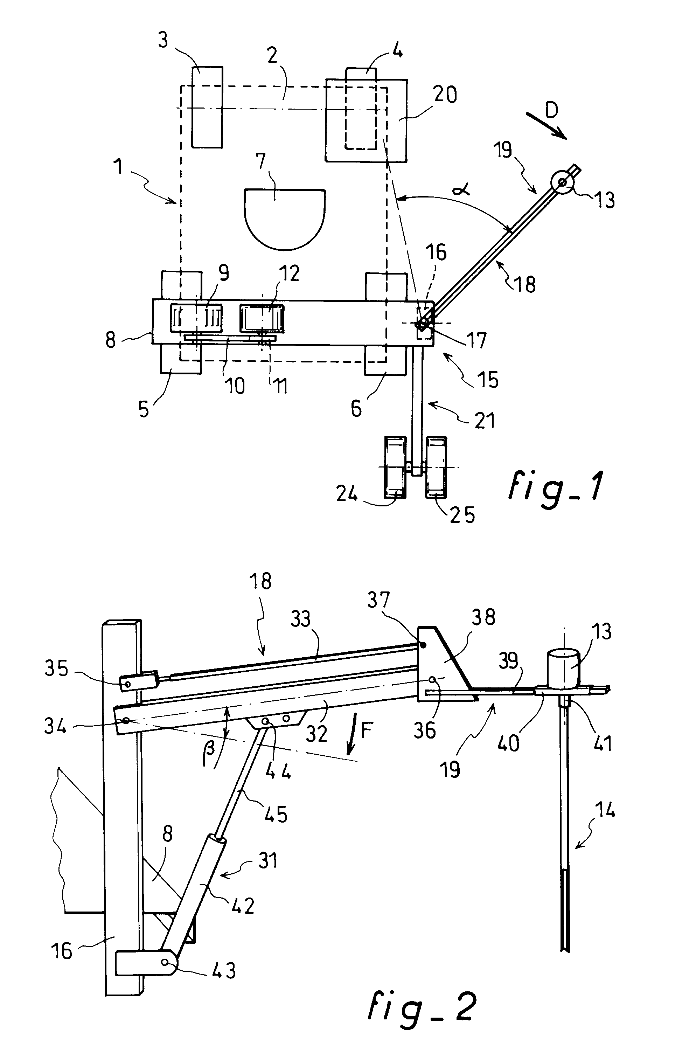

L'installation mobile 1 comprend un véhicule 2 léger tout terrain , représenté très schématiquement sur la figure 1 par un rectangle en pointillé , par les quatre roues motrices 3,4,5 et 6 et par le siège du conducteur 7.

Un véhicule 2 qui convient parfaitement est connu sous la marque YAMAHA , et la référence Y F M 350 BIG BEAR. Il s'agit d'un véhicule pesant de l'ordre de 250 kg , dont les roues sont équipées de pneus larges, basse pression, et qui permet un déplacement relativement rapide de l'ordre de 20km/h dans les champs.

Une traverse 8 est fixée rigidement sur le châssis du véhicule , au-dessus de l'amortisseur arrière. Cette traverse 8 supporte un moteur 9 à essence entraînant en rotation , grâce à une courroie 10, la roue 11 d'une pompe hydraulique 12 destinée à alimenter en fluide sous pression le moteur 13 d'entraînement en rotation de la sonde 14 de prélèvement , ainsi que les vérins hydrauliques qui seront décrits plus loin.

A une extrémité latérale 15 de la traverse 8, à droite sur la figure 1, est fixé un montant vertical 16. Le bras porteur 18 est solidaire du montant vertical 16 par un axe vertical de pivotement 17. L'extrémité libre 19 du bras porteur 18 supporte le moteur d'entraînement 13 et la sonde 14 de prélèvement.

A l'avant droit du châssis du véhicule 2, on a installé un bac 20 de réception des échantillons. Comme on le voit clairement sur la figure 1, le pivotement angulaire d'angle a dans le plan horizontal permet de déplacer le bras porteur 18 de sorte que la sonde 14 , avec l'échantillon de sol prélevé , soit au-dessus du bac 20.

L'ensemble compensateur 21 est fixé sous la traverse 8 vers son extrémité latérale 15. Cet ensemble compensateur 21 est composé d'un bras 22 qui est solidaire du châssis du véhicule 2 par un axe horizontal 23 de rotation. L'extrémité libre de ce bras 22 supporte un train de deux roues 24,25.

Un système amortisseur 26 , comportant un ressort de pression 27 , est fixé entre la traverse 8 et le bras 22, pivotant au niveau des deux goujons de fixation 28,29. Le ressort de pression 27 repousse le bras 22 et donc les roues 24,25 vers le sol 30.

La figure 2 représente un premier mode particulier de réalisation du bras porteur 18, supportant vers son extrémité libre 19 le moteur d'entraînement 13 et la sonde 14.

Le bras porteur 18 est un ensemble articulé qui comprend un premier sous-ensemble formant un parallélogramme déformable, un second sous-ensemble supportant le moteur 13 et la sonde, et enfin un vérin hydraulique 31 apte à déplacer le premier sous-ensemble.

Le premier sous-ensemble se compose d'une barre transversale 32 et d'une tige 33 de réglage qui sont fixées , pivotantes , par une extrémité respectivement 34,35 au montant vertical 16 et par l'autre extrémité respectivement 36,37 à une pièce de jonction 38 . La disposition des points de pivotement 34,35,36,37 , les longueurs de la barre transversale 32 et de la tige 33 sont telles que ce premier sous-ensemble forme un parallélogramme déformable , comme il sera expliqué ci-après.

Le second sous-ensemble comprend une plaque 39 fixée rigidement et horizontalement sur la pièce de jonction 38, et une cornière de coulissement 40 enserrant la plaque 39 et supportant le moteur hydraulique 13 d'entraînement en rotation de la sonde 14. L'embout 41 du moteur 13 dans lequel est emmanchée la sonde 14 passe à travers une encoche (non représentée sur la figure 2) pratiquée longitudinalement dans la plaque 39.

Le corps 42 du vérin hydraulique 31 est fixé pivotant au montant vertical 16 par son extrémité 43; l'extrémité 44 de la tige 45 du piston du vérin 31 est fixée, pivotante, sous la barre transversale 32.

Le fonctionnement de l'installation mobile 1 est le suivant. Lors du déplacement du véhicule 2, la tige 45 du vérin hydraulique 31 est entièrement sortie et le bras porteur 18 est placé sensiblement le long du véhicule 2.

Lorsque l'opérateur doit effectuer un prélèvement, le véhicule 2 étant à l'arrêt, l'opérateur met en marche le moteur à essence 8 et la pompe hydraulique 12 ; il commande le déplacement du bras porteur 18 d'un angle a dans la direction de la flèche D, de manière à placer la sonde 14 au-dessus de l'endroit convenable pour effectuer le prélèvement . Puis il actionne le vérin hydraulique 31 alimenté par la pompe hydraulique 12, de manière à ce que la tige 45 rentre dans le corps 42 du vérin 31. Ce déplacement de la tige 45 et de son extrémité 44 entraîne le déplacement angulaire d'angles dans un plan vertical dans le sens de la flèche F de la barre transversale 32. La barre 32 entraîne dans son déplacement d'angle la tige 33 et la pièce de jonction 38 dont elles sont toutes deux solidaires. La tige 33 et la barre 32 forment deux côtés égaux et parallèles d'un parallélogramme dont le montant vertical 16 et la pièce de jonction 38 sont les deux autres côtés . Ainsi dans son déplacement , la pièce de jonction 38 garde toujours la même direction , sensiblement verticale, la plaque 39 reste horizontale, et la sonde 14 reste verticale.

La sonde 14 est entraînée en rotation par le moteur 13. Lors du déplacement vertical décrit ci-dessus, la sonde 14 pénètre dans le sol jusqu'à une profondeur qui est fonction de la valeur de l'angle et prélève l'échantillon du sol.

La position de la sonde 14 correspond au point d'impact de celle-ci avec le sol. Au cours du déplacement angulaire de la pièce de jonction 38, la plaque 39 est amenée à coulisser dans la cornière 40 par rapport à la position fixe de la sonde 14. Le fait que le bras porteur 18 soit en porte-à-faux par rapport au véhicule 2 , puis le fonctionnement du vérin hydraulique 31 lors de la pénétration de la sonde 14 dans le sol mettent en jeu des forces qui déplacent le centre de gravité de l'installation mobile 1 et qui auraient tendance à déséquilibrer le véhicule 2 s'il n'y avait pas l'ensemble compensateur 21, dont le rôle est d'assurer la stabilité de l'installation 1 pendant les opérations de prélèvement en compensant le déséquilibre qu'elles provoquent.

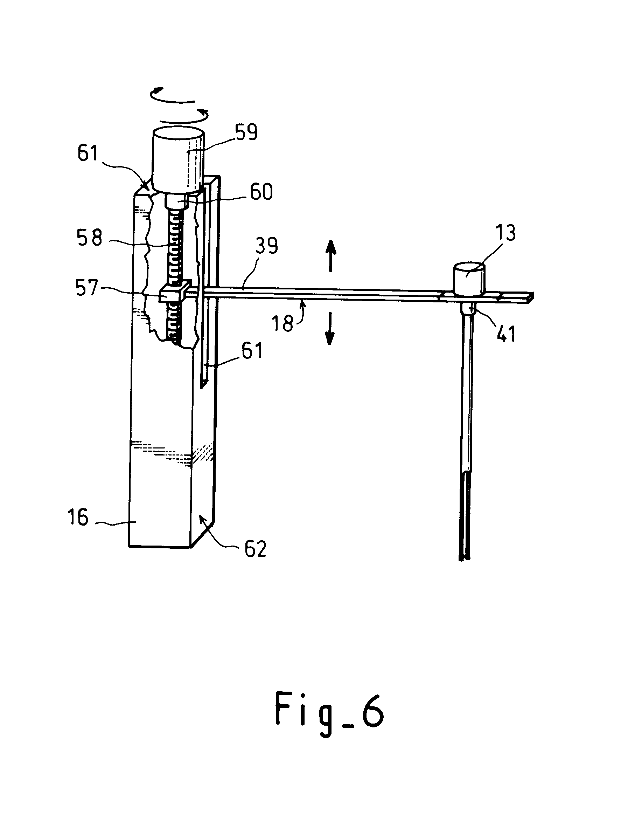

La figure 6 représente un deuxième mode particulier de réalisation du bras porteur 18 qui comprend également deux sous-ensembles, et qui diffère du premier mode de réalisation précité représenté à la figure 2, par son premier sous ensemble.

Ce premier sous-ensemble se compose d'une tige filetée 58 verticale, qui fait partie intégrante du montant vertical 16, et dont l'extrémité supérieure est dans l'embout 60 d'un moteur 59 qui est fixé sur la surface supérieure 61 du montant vertical 16; par conséquent, l'embout 60 passe à travers une encoche (non représentée sur la figure 6), pratiquée dans la surface supérieure 61. La plaque support 39 du deuxième sous-ensemble, qui est identique à celui décrit dans le premier mode particulier de réalisation du bras porteur 18, est fixée horizontalement à un écrou 57, lequel est traversé par la tige filetée 58. A cet effet, la plaque support 39 passe à travers une fente 61 de section, légèrement supérieure , pratiquée verticalement dans la face du montant vertical 16.

Ainsi, lorsque la sonde 14 est positonnée au-dessus de l'endroit convenable pour effectuer le prélèvement, le positionnement s'effectuant de la même façon que dans le premier mode de réalisation, l'opérateur met en marche le moteur 59, qui entraîne en rotation, la tige filetée 58.

La plaque support 39 faisant contre poids empêche l'ecrou 57 d'entrer en rotation. Celui-ci possède alors un mouvement de translation selon un axe vertical, le long de la tige filetée 59.

Le sens de translation dépend du sens de rotation du moteur et du sens du filetage de la tige. La rotation de la tige filetée 59, a donc pour conséquence le déplacement vers le haut ou vers le bas, de la plaque support 39 qui est solidaire de l'écrou et qui reste horizontale. Ce déplacment est en outre limité à une zone correspondant à la fente 61, qui sert de guide pour la plaque support 39. La tige filetée 58 peut n'être filetée que sur la partie correspondant à la zone en regard de la fente 61.

Le déplacement vertical de la plaque support 39 entraîne le même déplacement vertical de la sonde 14, qui est par ailleurs entrainée en rotation par le moteur 13.

Dans un mode de réalisation équivalent , on pourrait, sans sortir du cadre de l'invention, envisager de fusionner l'écrou 57 et la plaque support 39 en une seule pièce, dont l'extrémité percée et taraudée, serait traversée par la tige filetée 58.

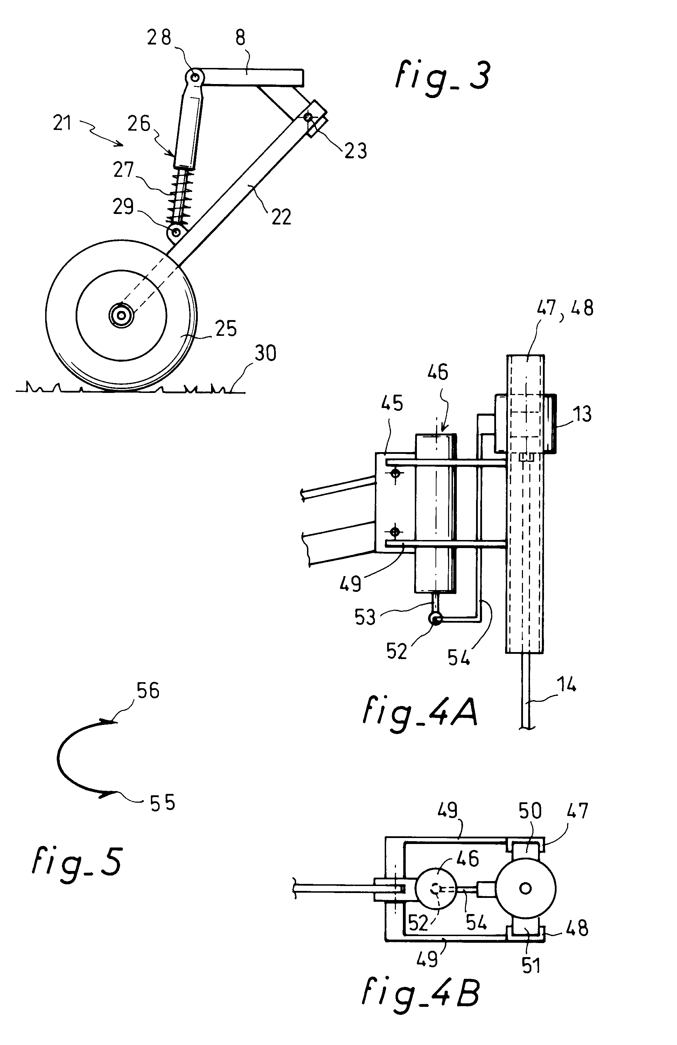

Sur les figures 4A et 4B , on a représenté un autre mode de réalisation utilisé notamment pour les prélèvements à grandes profondeurs , par exemple de 90cm pour les dosages de reliquat d'azote dans le sol.

Par rapport à ce qui a été décrit concernant le premier mode particulier de réalisation, seul diffère le second sous-ensemble, qui est fixé à la pièce de jonction 45 du premier sous-ensemble.

Dans ce cas, le second sous-ensemble est composé d'un vérin hydraulique de pénétration 46, d'un jeu de glissières de maintien 47,48, de la sonde 14 et de son moteur d'entraînement 13.

Le vérin de pénétration 46 est fixé à la plaque de jonction 45.

Les deux glissières de maintien 47,48 sont verticales et fixées à la plaque de jonction 45 par des entretoises 49; elles ont une section transversale en U, les branches du U se faisant face.

Le moteur d'entraînement 13 en rotation de la sonde 14 comporte deux épaulements radiaux 50,51. Il est positionné entre les deux glissières 47,48 ; chacun des épaulements 50,51 étant conformé pour se placer dans le logement intérieur constitué par le U de la glissière correspondante.

L'extrémité libre 52 de la tige 53 du vérin de pénétration 46 est reliée au moteur 13 d'entraînement en rotation de la sonde 14 par une pièce coudée de liaison 54. Comme on peut le voir sur la figure 4A, le moteur 13 d'entraînement se trouve placé au niveau voire au-dessus du corps du vérin 46 de pénétration.

Le fonctionnement de ce second sous-ensemble est le suivant. La pénétration de la sonde 14 dans le sol se fait en deux temps. La tige 53 du vérin de pénétration 46 étant en position rentrée, le déplacement vertical de la sonde se fait dans le premier temps par l'action du premier vérin 31 provoquant le déplacement angulaire (angle Béta) du bras 18. La sonde 14 en rotation pénètre dans le sol d'environ 40cm. Puis dans le deuxième temps, le vérin de pénétration 46 est actionné , la tige 53 sort progressivement du corps du vérin 46, entraînant par la pièce de liaison 54, le moteur 13, qui coulisse verticalement par les épaulements radiaux 50,51 dans les glissières de maintien 47,48.

Le mode de réalisation utilisé notamment pour les prélèvements à grandes profondeurs, qui vient d'être décrit, peut en outre s'appliquer de la même façon au bras porteur tel qu'il a été décrit dans le deuxième mode particulier de réalisation :
Cette application n'est pas représentée, mais est à la portée de l'homme du métier qui adaptera aisément le second sous-ensemble composé notamment d'un vérin hydraulique de pénétration 46, et d'un jeu de glissières de maintien 47, 48 et représenté sur les figures 4 A et 4 B, au bras porteur 18 selon son deuxième mode de réalisation représenté à la figure 6.

La sonde 14 de prélèvement peut être une sonde traditionnelle. De meilleurs résultats ont été obtenus avec une sonde dont la partie d'attaque du sol a une section transversale ayant le profil représenté à la figure 5. Il s'agit sensiblement d'une demi ellipse évidée dont les bords libres légèrement repliés forment une arête acérée.

L'invention n'est pas limitée au mode de réalisation qui a été décrit de manière non exhaustive mais en couvre toutes les variantes. En particulier, l'ensemble compensateur 21 peut mettre en oeuvre des moyens équivalents à ceux décrits, par exemple des moyens qui n'interviendraient que pendant le déroulement des opérations de prélèvements.

## Revendications

1. Installation mobile (1) de prélèvement d'échantillons de sol comportant un véhicule (2) sur lequel est montée une sonde (14) de prélèvement entraînable en rotation et en translation verticale caractérisée en ce que le véhicule (2) étant un véhicule léger, la sonde (14) est montée à l'extrémité d'un bras porteur (18) pivotant autour d'un axe (17) solidaire du châssis du véhicule (2), et en ce qu'elle comprend un ensemble compensateur (21), solidaire du châssis du véhicule (2) et apte à s'appliquer sur le sol (30) au moins pendant le prélèvement en sorte d'assurer la stabilité du véhicule (2)

2. Installation selon la revendication 1 caractérisée en ce que le véhicule léger est un véhicule tout terrain à quatre roues motrices, ayant des pneus larges basse pression, dont le poids normal est de l'ordre de 250kg.

3. Installation selon la revendication 1 caractérisée en ce que l'ensemble compensateur (21) comprend au moins une roue complémentaire (24,25) , non motrice, montée à l'extrémité libre d'un bras (22) solidaire du châssis du véhicule (2) et en ce qu'elle comporte des moyens de pression (26) , aptes à repousser ladite roue (24,25) vers le sol (30).

4. Installation selon l'une des revendications 1 ou 3 caractérisée en ce que la zone d'application sur le sol (30) de l'ensemble compensateur (21) est extérieure au polygone normal de sustentation du véhicule (2) et située du côté du bras porteur (18) de la sonde (14).

5. Installation selon la revendication 1 caractérisée en ce qu'elle comporte un bac de réception (20) des échantillons , qui est fixé sur le châssis du véhicule (2) à une distance de l'axe de pivotement (17) du bras porteur (18) qui est sensiblement égale à la longueur dudit bras (18).

6. Installation selon la revendication 1 caractérisée en ce que le bras porteur (18) comporte : a. une plaque support (39) , horizontale, sur laquelle sont montés coulissants la sonde (14) et au moins le moteur (13) d'entraînement en rotation de la sonde (14) ;
b. une pièce de jonction (38) sur laquelle est fixée rigidement ladite plaque support (39) ;
c. deux tiges parallèles (32,33) , de même longueur, dont les extrémités (34,35,36,37) sont solidaires d'une part du montant vertical (16) constituant l'axe de pivotement du bras porteur (18) et d'autre part de la pièce de jonction (38) , de sorte que l'ensemble forme un parallélogramme déformable ;
d. un vérin (31) dont le corps (42) est solidaire du montant vertical ( 16) et dont l'extrémité (44) de la tige (45) du piston est solidaire d'une des deux tiges (32,33) formant le parallélogramme déformable.

7. Installation selon la revendication 1 caractérisée en ce qu'elle comporte une tige filetée (58), entraînée en rotation par un moteur (59) et incorporée dans un montant vertical (16) qui constitue l'axe de pivotement (17) du bras porteur (18) et qui comporte une fente verticale (61), et en ce que le bras porteur (18) comporte une plaque support, horizontale, sur laquelle sont montés coulissantes vers une extrémité, la sonde (14) et au moins le moteur (18) d'entraînement en rotation de la sonde (18) et dont l'autre extrémité, traversant le montant (16) par la fente (61), est solidaire d'un écrou (57) traversé par la tige filetée (58).

8. Installation selon l'une des revendications 1, 6 ou 7 caractérisée en ce qu'elle comporte un vérin (46) de pénétration dont le corps est fixé à l'extrémité libre du bras porteur (18), des glissières de maintien (47,48) qui sont fixées verticales à l'extrémité libre du bras porteur (18) et le long desquelles peuvent coulisser la sonde (14) et son moteur d'entraînement en rotation (13) et en ce que l'extrémité libre de la tige (53) du vérin de pénétration (46) est reliée par une pièce de liaison (54) au moteur d'entraînement (13), en sorte que l'action du vérin (46) provoque le déplacement vertical de la sonde (14) dans les glissières de maintien (47,48).

9. Installation selon la revendication 1 caractérisée en ce que la partie d'attaque de la sonde (14) a en section transversale un profil sensiblement semi-elliptique dont les bords libres (55,56) repliés forment des arêtes acérées.
